(19) Europäisches Patentamt / European Patent Office / Office européen des brevets

(11) **EP 3 747 423 B1**

(12) **EUROPEAN PATENT SPECIFICATION**

(45) Date of publication and mention
of the grant of the patent:
**03.05.2023  Bulletin 2023/18**

(21) Application number: **20176474.3**

(22) Date of filing: **26.05.2020**

(51) International Patent Classification (IPC):
**A61K 8/31** *(2006.01)*      **A61K 47/06** *(2006.01)*
**A61Q 19/00** *(2006.01)*

(52) Cooperative Patent Classification (CPC):
**A61K 8/31; A61Q 19/00;** A61K 2800/10;
A61K 2800/5922

(54) **HYDROCARBON SOLVENT COMPOSITION**

KOHLENWASSERSTOFFLÖSUNGSMITTELZUSAMMENSETZUNG

COMPOSITION DE SOLVANT HYDROCARBONÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **03.06.2019  KR 20190065479**

(43) Date of publication of application:
**09.12.2020  Bulletin 2020/50**

(73) Proprietors:
 • **SK Innovation Co., Ltd.
   Seoul 03188 (KR)**
 • **SK Geo Centric Co., Ltd.
   Jongno-gu
   Seoul 03188 (KR)**

(72) Inventor: **CHOI, Jeong Eop
34124 Daejeon (KR)**

(74) Representative: **Behr, Wolfgang
Lorenz Seidler Gossel
Rechtsanwälte Patentanwälte
Partnerschaft mbB
Widenmayerstraße 23
80538 München (DE)**

(56) References cited:
**EP-A1- 3 228 309      WO-A2-2015/101837
JP-A- H07 228 897      JP-A- 2000 044 994**

**Description**

**CROSS-REFERENCE TO RELATED APPLICATIONS**

**TECHNICAL FIELD**

The following disclosure relates to a hydrocarbon solvent composition.

**BACKGROUND**

A hydrocarbon solvent is used as an industrial cleaning solvent, a solvent for polymerizing natural and synthetic resins, a solvent for adhesives and tapes, and a solvent for drug reactions.
A hydrocarbon solvent used for cleaning in the production and use of an electronic apparatus should have sufficient dissolving power in order to remove impurities, have an excellent drying property for removal after the cleaning, and have low harmfulness to a human body.
To this end, the hydrocarbon solvent should have an aniline point, which is an indirect measure of the dissolving power, low enough for cleaning, and have a sufficient drying property. To this end, it is necessary to adjust components included in the hydrocarbon solvent and the contents of the components.
In addition, in order to reduce the harmfulness to the human body, it is necessary to remove an aromatic component which causes odor and is harmful to the human body.

**SUMMARY**

An embodiment of the present invention is directed to providing a hydrocarbon solvent composition having excellent dissolving power and drying property, no odor, and low harmfulness to a human body.
In one general aspect, a hydrocarbon solvent composition includes: 80 wt% or more of a $C_7$ saturated hydrocarbon component and 1 wt% or less of an aromatic component, based on the total weight of the hydrocarbon solvent composition, wherein the hydrocarbon solvent composition satisfies the following Equation 1:

[Equation 1]

$$5 < \frac{W_{IC7}}{W_{NC7}} < 100$$

wherein $W_{IC7}$ is a content of $C_7$ isoparaffin, based on the total weight of the hydrocarbon solvent composition, and $W_{NC7}$ is a content of $C_7$ n-paraffin, based on the total weight of the hydrocarbon solvent composition.
The hydrocarbon solvent composition may further include 1 wt% or less of a $C_5$ saturated hydrocarbon component, based on the total weight of the hydrocarbon solvent composition.
The hydrocarbon solvent composition may further include 1 wt% or less of a $C_8$ saturated hydrocarbon component, based on the total weight of the hydrocarbon solvent composition.
The hydrocarbon solvent composition may further include 30 wt% or more and 45 wt% or less of a $C_7$ cyclic saturated hydrocarbon component, based on the total weight of the hydrocarbon solvent composition.
The hydrocarbon solvent composition may further include 5 wt% or less of $C_7$ n-paraffin, based on the total weight of the hydrocarbon solvent composition.
The hydrocarbon solvent composition may further satisfy the following Equation 2:

[Equation 2]

$$1 \leq \frac{W_{IC}}{W_N} \leq 1.4$$

wherein $W_{IC}$ is a content of isoparaffin, based on the total weight of the hydrocarbon solvent composition, and $W_N$ is a content of a cyclic saturated hydrocarbon component, based on the total weight of the hydrocarbon solvent composition.

The hydrocarbon solvent composition may further include less than 1 ppm of sulfur, based on the total weight of the hydrocarbon solvent composition.

The hydrocarbon solvent composition may include 80 wt% or more and 99.5 wt% or less of the $C_7$ saturated hydrocarbon component, 0.0001 wt% or more and 1 wt% or less of the aromatic component, and 0.4 wt% or more and 19 wt% or less of the remaining components other than the $C_7$ saturated hydrocarbon component and the aromatic component, based on the total weight of the hydrocarbon solvent composition.

The hydrocarbon solvent composition may include 80 wt% or more and 99.5 wt% or less of the $C_7$ saturated hydrocarbon component, 0.0001 wt% or more and 1 wt% or less of the aromatic component, 0.001 wt% or more and 1 wt% or less of a $C_5$ saturated hydrocarbon component, 0.001 wt% or more and 1 wt% or less of a $C_8$ saturated hydrocarbon component, and 0.1 wt% or more and 17 wt% or less of a $C_6$ saturated hydrocarbon component, based on the total weight of the hydrocarbon solvent composition.

The hydrocarbon solvent composition may have an aniline point of 70°C or less.

The hydrocarbon solvent composition may have an evaporation rate of 25 to 45 when an evaporation rate of n-butyl acetate (n-BuAc) is set to 100 according to ASTM D3539.

Other features and aspects will be apparent from the following detailed description and the claims.

## DETAILED DESCRIPTION OF EMBODIMENTS

Unless otherwise defined, all terms (including technical and scientific terms) used herein have the general meaning understood by those skilled in the art to which the present invention pertains. Throughout the present specification, unless described to the contrary, "comprising" any component will be understood to imply the further inclusion of other elements rather than the exclusion of other elements. In addition, unless explicitly described to the contrary, a singular form includes a plural form in the present specification.

"%" described herein means wt%, unless defined otherwise.

An aspect of the present invention relates to a hydrocarbon solvent composition having excellent dissolving power and drying property, no odor, and low harmfulness to a human body, the hydrocarbon solvent composition including 80 wt% or more of a $C_7$ saturated hydrocarbon component and 1 wt% or less of an aromatic component, based on the total weight of the hydrocarbon solvent composition, wherein the hydrocarbon solvent composition satisfies the following Equation 1:

[Equation 1]

$$5 < \frac{W_{IC7}}{W_{NC7}} < 100$$

wherein $W_{IC7}$ is a content of $C_7$ isoparaffin, based on the total weight of the hydrocarbon solvent composition, and $W_{NC7}$ is a content of $C_7$ n-paraffin, based on the total weight of the hydrocarbon solvent composition.

The hydrocarbon solvent composition according to an aspect of the present invention includes 80 wt% or more of the $C_7$ saturated hydrocarbon component, and a ratio between $C_7$ isoparaffin and $C_7$ n-paraffin (i.e., n-heptane) satisfies the above Equation 1. Therefore, the hydrocarbon solvent composition including a large amount of the $C_7$ saturated hydrocarbon component commonly used as an industrial solvent such as an industrial cleaning solvent, excellent dissolving power for an organic material, and an excellent drying property, may be provided.

In addition, the hydrocarbon solvent composition includes an excessive amount of $C_7$ isoparaffin compared to $C_7$ n-paraffin, such that a smell of the solvent may become mild and an evaporation rate of the solvent may become excellent.

In addition, the hydrocarbon solvent composition includes a small amount of n-hexane, which is harmful to the human body by causing abnormality in a central nervous system and 1 wt% or less of an aromatic component, which is a carcinogenic substance, and may thus have low harmfulness to the human body and low odor, such that safety of the hydrocarbon solvent composition in use may be improved.

Accordingly, the hydrocarbon solvent composition may be used as an industrial cleaning solvent, a solvent for

$$\frac{W_{IC7}}{W_{NC7}}$$

polymerizing natural and synthetic resins, a solvent for adhesives and tapes, and a solvent for drug reactions.

in Equation 1 may be, but is not necessarily limited to, more specifically, 10 or more and 80 or less, and more further specifically, 20 or more and 60 or less, 40 or more and 80 or less, or 50 or more and 70 or less.

The hydrocarbon solvent composition according to an aspect of the present invention may include, but is not necessarily limited to, 10 wt% or more and 60 wt% or less of $C_7$ isoparaffin and 0.1 wt% or more and 5 wt% or less of $C_7$ n-paraffin, based on the total weight of the hydrocarbon solvent composition, in the range satisfying Equation 1.

The hydrocarbon solvent composition according to an aspect of the present invention may include more specifically, 30 wt% or more and 60 wt% or less of $C_7$ isoparaffin and 0.5 wt% or more and 3 wt% or less of $C_7$ n-paraffin, and more further specifically, 50 wt% or more and 55 wt% or less of $C_7$ isoparaffin and 0.8 wt% or more and 1 wt% or less of $C_7$ n-paraffin, based on the total weight of the hydrocarbon solvent composition.

Equation 1 is satisfied and a content of $C_7$- normal paraffin satisfies the range described above, such that a smell of the solvent may become mild, an evaporation rate of the solvent may become excellent, and harmfulness to the human body and odor of the solvent may be reduced.

The hydrocarbon solvent composition according to an aspect of the present invention may include a sulfur component that inevitably remains in a preparation process. A content of the sulfur component may be less than 1 ppm, based on the total weight of the hydrocarbon solvent composition.

The content of the sulfur component, which generates a unique odor, included in the hydrocarbon solvent composition is less than 1 ppm, which is low, such that the odor of the hydrocarbon solvent composition may be reduced.

The hydrocarbon solvent composition according to an aspect of the present invention may include 80 wt% or more and 99.5 wt% or less of the $C_7$ saturated hydrocarbon component, 0.0001 wt% or more and 1 wt% or less of the aromatic component, and 0.4 wt% or more and 19 wt% or less of the remaining components other than the $C_7$ saturated hydrocarbon component and the aromatic component, based on the total weight of the hydrocarbon solvent composition.

Here, the remaining components other than the $C_7$ hydrocarbon component and the aromatic component may be a $C_5$ saturated hydrocarbon component, a $C_6$ saturated hydrocarbon component, and a $C_8$ saturated hydrocarbon component.

The hydrocarbon solvent composition according to an aspect of the present invention may include, specifically, 1 wt% or less of the $C_5$ saturated hydrocarbon component, and more specifically, 0.5 wt% or less, 0.1 wt% or less, or 0.01 wt% or less of the $C_5$ saturated hydrocarbon component, based on the total weight of the hydrogen solvent composition.

The hydrocarbon solvent composition according to an aspect of the present invention includes a very small amount of the $C_5$ saturated hydrocarbon component, which has a relatively large ignitability compared to the other hydrocarbon components, or does not substantially include the $C_5$ saturated hydrocarbon component. Thus, when the hydrocarbon solvent composition is used as an industrial cleaning solvent, a solvent for polymerizing natural and synthetic resins, a solvent for adhesives and tapes, a solvent for drug reactions, or the like, a fire risk may be greatly reduced, such that safety may be improved.

The hydrocarbon solvent composition according to an aspect of the present invention may include 1 wt% or less of the $C_8$ saturated hydrocarbon component, and more specifically, 0.5 wt% or less, or 0.3 wt% or less of the $C_8$ saturated hydrocarbon component, based on the total weight of the hydrocarbon solvent composition.

The $C_8$ saturated hydrocarbon component has an evaporation rate slower than that of a saturated hydrocarbon component having relatively smaller carbon atoms. Therefore, when a large amount of the $C_8$ saturated hydrocarbon component is included in the solvent, the evaporation rate of the solvent is reduced, such that a drying property of the solvent may be lowered.

The hydrocarbon solvent composition according to an aspect of the present invention includes a very small amount of the $C_8$ saturated hydrocarbon component or does not substantially include the $C_8$ saturated hydrocarbon component. Therefore, when the hydrocarbon solvent is used as an industrial cleaning solvent, a solvent for polymerizing natural and synthetic resins, a solvent for adhesives and tapes, a solvent for drug reactions or the like, the solvent may have a sufficient drying property.

The hydrocarbon solvent composition according to an aspect of the present invention may include 30 wt% or more and 45 wt% or less of a $C_7$ cyclic saturated hydrocarbon component ($C_7$ naphthene component) in the $C_7$ saturated hydrocarbon component, and more specifically, 35 wt% or more and 45 wt% or less of a $C_7$ cyclic saturated hydrocarbon component ($C_7$ naphthene component) in the $C_7$ saturated hydrocarbon component, based on the total weight of the hydrocarbon solvent composition.

The hydrocarbon solvent composition according to an aspect of the present invention includes such a content of $C_7$ cyclic saturated hydrocarbon component, such that excellent dissolving power of the cyclic saturated hydrocarbon may be sufficiently utilized. Thus, excellent dissolving power of the hydrocarbon solvent composition may be implemented.

Meanwhile, the hydrocarbon solvent composition according to an aspect of the present invention may further satisfy the following Equation 2:

[Equation 2]

$$1 \leq \dfrac{W_{IC}}{W_N} \leq 1.4$$

wherein $W_{IC}$ is a content of isoparaffin, based on the total weight of the hydrocarbon solvent composition, and $W_N$ is a content of a cyclic saturated hydrocarbon component, based on the total weight of the hydrocarbon solvent composition.

Since the cyclic saturated hydrocarbon has dissolving power more excellent than that of isoparaffin, but may cause odor and be harmful to the human body, the odor and the harmfulness to the human body need to be adjusted to be maintained at an appropriate level or less while implementing sufficient dissolving power and a drying property.

The content of the isoparaffin and cyclic saturated hydrocarbon components included in the hydrocarbon solvent composition according to an aspect of the present invention satisfy Equation 2, such that it is possible to utilize the excellent dissolving power of naphthene and the drying property of isoparaffin while adjusting the smell and the harmfulness to an appropriate level.

The hydrocarbon solvent composition according to an aspect of the present invention may include, but is not necessarily limited to, 40 wt% or more and 60 wt% or less of isoparaffin and 40 wt% or more and 60 wt% or less of the cyclic saturated hydrocarbon, based on the total weight of the hydrocarbon solvent composition, in a range in which the above Equation 2 is satisfied. More specifically, the hydrocarbon solvent composition according to an aspect of the present invention may include 45 wt% or more and 55 wt% or less of isoparaffin and 45 wt% or more and 55 wt% or less of the cyclic saturated hydrocarbon, based on the total weight of the hydrocarbon solvent composition.

The hydrocarbon solvent composition according to an aspect of the present invention may include 80 wt% or more and 99.5 wt% or less of the $C_7$ saturated hydrocarbon component, 0.0001 wt% or more and 1 wt% or less of the aromatic component, 0.001 wt% or more and 1 wt% or less of the $C_5$ saturated hydrocarbon component, 0.001 wt% or more and 1 wt% or less of the $C_8$ saturated hydrocarbon component, and 0.1 wt% or more and 17 wt% or less of the $C_6$ saturated hydrocarbon component, based on the total weight of the hydrocarbon solvent composition.

More specifically, the hydrocarbon solvent composition according to an aspect of the present invention may include 85 wt% or more and 99.5 wt% or less of the $C_7$ saturated hydrocarbon component, 0.0001 wt% or more and 0.1 wt% or less of the aromatic component, 0.001 wt% or more and 0.5 wt% or less of the $C_5$ saturated hydrocarbon component, 0.001 wt% or more and 0.5 wt% or less of the $C_8$ saturated hydrocarbon component, and 0.1 wt% or more and 14 wt% or less of the $C_6$ saturated hydrocarbon component, based on the total weight of the hydrocarbon solvent composition.

More further specifically, the hydrocarbon solvent composition according to an aspect of the present invention may include 94 wt% or more and 99.5 wt% or less of the $C_7$ saturated hydrocarbon component, 0.0001 wt% or more and 0.1 wt% or less of the aromatic component, 0.001 wt% or more and 0.5 wt% or less of the $C_5$ saturated hydrocarbon component, 0.001 wt% or more and 0.5 wt% or less of the $C_8$ saturated hydrocarbon component, and 0.1 wt% or more and 5 wt% or less of the $C_6$ saturated hydrocarbon component, based on the total weight of the hydrocarbon solvent composition.

In the hydrocarbon solvent composition according to an aspect of the present invention, the $C_7$ saturated hydrocarbon component may include, but is not particularly limited to, any one or two or more mixtures selected from the group consisting of 2,2,3-trimethylbutane, 2,2-dimethylpentane, 2,3-dimethylpentane, 2,4-dimethylpentane, 3,3-dimethylpentane, 3-ethylpentane, 2-methylhexane, 3-methylhexane, n-heptane, ethylcyclopentane, methylcyclohexane, 1,1-dimethylcyclopentane, lc,3-dimethylcyclopentane, lt,3-dimethylcyclopentane, and 2t,2-dimethylcyclopentane.

In the $C_7$ saturated hydrocarbon component, isoparaffin may include, but is not particularly limited to, any one or two or more mixtures selected from the group consisting of 2,2,3-trimethylbutane, 2,2-dimethylpentane, 2,3-dimethylpentane, 2,4-dimethylpentane, 3,3-dimethylpentane, 3-ethylpentane, 2-methylhexane, and 3-methylhexane.

In the C7 saturated hydrocarbon component, the cyclic saturated hydrocarbon may include, but is not particularly limited to, any one or two or more mixtures selected from the group consisting of ethylcyclopentane, methylcyclohexane, 1,1-dimethylcyclopentane, lc,3-dimethylcyclopentane, lt,3-dimethylcyclopentane, and 2t,2-dimethylcyclopentane.

In the hydrocarbon solvent composition according to an aspect of the present invention, the $C_6$ saturated may include, but is not particularly limited to, any one or two or more mixtures selected from the group consisting of 2-methylpentane, 3-methylpentane, 2,2-dimethylbutane, 2,3-dimethylbutane, cyclohexane, n-hexane, and 1-methylcyclopentane.

In the hydrocarbon solvent composition according to an aspect of the present invention, the $C_8$ saturated hydrocarbon may include, but is not particularly limited to, any one or two or more mixtures selected from the group consisting of 2,2,4-trimethylpentane, 2,3,4-trimethylpentane, n-octane, 1,1-dimethylcyclohexane, 1,3-dimethylcyclohexane, and ethylcyclohexane.

In the hydrocarbon solvent composition according to an aspect of the present invention, the aromatic component may include, but is not particularly limited to, any one or two or more mixtures selected from the group consisting of benzene, toluene, xylene, naphtalene, and anthracene.

A mixed aniline point of the hydrocarbon solvent composition according to an aspect of the present invention when measured according to ASTM D611 may be 70°C or less. A low mixed aniline point means high dissolving power for an organic material, and the hydrocarbon solvent composition according to an aspect of the present invention has a mixed aniline point of 70°C or less, and more specifically, 50°C or less, and may thus have excellent dissolving power.

The hydrocarbon solvent composition according to an aspect of the present invention may have an evaporation rate of 25 or more and 45 or less when an evaporation rate of n-butyl acetate (n-BuAc) is set to 100 according to ASTM D3539. The hydrocarbon solvent composition according to an aspect of the present invention may have a high evaporation power, and may thus be adopted as an industrial solvent such as an industrial cleaning solvent.

The hydrocarbon solvent composition according to an aspect of the present invention may have a distillation range in which an initial boiling point is 60°C or more and a dry point is 105°C or less.

The hydrocarbon solvent composition according to an aspect of the present invention may be produced from, for example, naphtha used as a raw material of a benzene-toluene-xylene process (BTX process) for producing benzene, toluene, and xylene.

Specifically, the hydrocarbon solvent composition may be produced by extracting a $C_7$ component by distillation from naphtha subjected to a desulfurization process commonly known as a pre-treatment process of an aromatic treatment process, removing the aromatic component through a hydrogenation process, and removing the n-paraffin through an adsorption process using a simulated moving bed (SMB) or the like.

However, a method of producing the hydrocarbon solvent composition according to an aspect of the present invention is not limited thereto, and may be a method by which those skilled in the art may easily produce the hydrocarbon solvent composition, such as a method of blending corresponding components with each other so as to satisfy the contents of the hydrocarbon solvent composition according to an aspect of the present invention.

Hereinafter, Examples of the present invention and Comparative Examples will be described. However, the following Examples are only examples of the present invention, and the present invention is not limited thereto.

**[Examples and Comparative Examples]**

The hydrocarbon solvent was produced by removing component of $C_6$ or less and $C_8$ or more through distillation using desulfurized naphtha subjected to a desulfurization process, as a raw material, adding the resultant to a hydrogenation reactor to remove an aromatic component, and then removing an n-paraffin component using an SMB (Example 1).

A composition of a hydrocarbon solvent of Example 1 is as shown in Table 1 below.

In addition, in order to evaluate a degree of odor of the solvent of Example 1, a solvent (Comparative Example 1) having compositions different from those of Example 1 and a naphtha oil (Comparative Example 2), that is, a $C_6$-$C_{10}$ aliphatic oil, having a boiling point range of 93 to 156°C and has a sulfur content of 2.5 ppm was prepared.

The solvent of Comparative Example 1 was produced by blending a corresponding classification of hydrocarbon components with each other, and a detailed composition thereof is as shown in Table 2.

[Table 1]

| | Classification 1 | Content (wt%) | Classification 2 | Content (wt%) | Equation | Value |
|---|---|---|---|---|---|---|
| Example 1 | C$_5$ saturated hydrocarbon | 0.005 | - | - | - | - |
| | C$_6$ saturated hydrocarbon | 0.597 | - | - | - | - |
| | C$_7$ saturated hydrocarbon | 99.163 | Isoparaffin | 54.089 (W$_{IC7}$) | W$_{IC7}$/W$_{NC7}$ | 59.569 |
| | | | N-paraffin (n-heptane) | 0.908 (W$_{NC7}$) | | |
| | | | Cyclic saturated hydrocarbon | 44.166 | - | - |
| | C$_8$ saturated hydrocarbon | 0.235 | - | - | - | - |
| | Aromatic | < 0.001 wt% | - | - | - | - |
| | Sulfur | < 1 ppm | - | - | - | - |
| | Isoparaffin | 54.351 (W$_{IC}$) | - | - | W$_{IC}$/W$_N$ | 1.215 |
| | Cyclic saturated hydrocarbon | 44.741 (W$_N$) | - | - | | |
| | N-paraffin | 0.908 | - | - | - | - |

[Table 2]

| | Classification 1 | Content (wt%) | Classification 2 | Content (wt%) | Equation | Value |
|---|---|---|---|---|---|---|
| Comp. Example 1 | C$_5$ saturated hydrocarbon | 0.01 | - | - | - | - |
| | C$_6$ saturated hydrocarbon | 0.15 | - | - | - | - |
| | C$_7$ saturated hydrocarbon | 99.8 | Isoparaffin | 28.8 (W$_{IC7}$) | W$_{IC7}$/W$_{NC7}$ | 1.44 |
| | | | N-paraffin (n-heptane) | 20 (W$_{NC7}$) | | |
| | | | Cyclic saturated hydrocarbon | 51 | - | - |
| | C$_8$ saturated hydrocarbon | 0.04 | - | - | - | - |
| | Aromatic | < 0.001 wt% | - | - | - | - |
| | Sulfur | < 1 ppm | - | - | - | - |

(continued)

| | Classificat ion 1 | Content (wt%) | Classification 2 | Content (wt%) | Equation | Value |
|---|---|---|---|---|---|---|
| | Isoparaffin | 28.9 ($W_{IC}$) | - | - | $W_{IC}/W_N$ | 0.57 |
| | Cyclic saturated hydrocarbon | 51 ($W_N$) | - | - | | |
| | N-paraffin | 20.1 | - | - | - | - |

**[Experimental Example 1]**

Physical properties for the solvents of Example 1 were measured as follows and are listed in Table 3.

1. Mixed aniline point

A mixed aniline point was measured according to ASTM D611.

2. Distillation range (IBP-DP)

A range from an initial boiling point (IBP) to a drying point (DP) was measured according to ASTM D86.

3. Evaporation rate

An evaporation rate was measured according to ASTM D3539, and when an evaporation rate of n-butyl acetate (n-BuAc) is set to 100 according to ASTM D3539, an evaporation rate of the solvent was measured.

[Table 3]

| Aniline point (°C) | Distillation range (°C, IBP ~ DP) | Evaporation rate |
|---|---|---|
| 35°C | 75~98 | 40 |

**[Experimental Example 2]**

In order to evaluate the odor for the solvent of Example 1, a survey evaluation of the solvents of Example 1, Comparative Example 1, and Comparative Example 2 was conduced on ten researchers in a petrochemical technical field.
Specifically, 10 mL of each solvent was prepared, and the researchers were allowed to smell each solvent for two seconds and were allowed to give scores to each solvent according to the following criteria:

3 points: no repulsion
2 points: moderate repulsion
1 point: serious repulsion

Averages of evaluation scores for each solvent are listed in Table 4.

[Table 4]

| Example 1 | Comp. Example 1 | Comp. Example 2 |
|---|---|---|
| 2.8 | 2.1 | 1.3 |

It could be confirmed from Tables 3 and 4 that the hydrocarbon solvent according to the present invention has an aniline point of 35°C, which means excellent dissolving power, and has an excellent evaporation rate and low odor.
In addition, since the hydrocarbon solvent according to the present invention has very small amounts of aromatic and n-paraffin components, the aromatic and normal paraffin components are substantially removed. Therefore, the hydrocarbon solvent has no odor and has very low harmfulness to the human body.

Further, the hydrocarbon solvent according to the present invention includes a small amount of $C_8$ saturated hydrocarbon, such that a drying property is excellent.

Further, the hydrocarbon solvent composition includes 30 wt% or more and 45 wt% or less of the $C_7$ cyclic saturated hydrocarbon component based on the total weight of the hydrocarbon solvent composition, and may thus exhibit excellent dissolving power, and the isoparaffin component and the cyclic hydrocarbon component are maintained in an appropriate ratio, such that harmfulness of the hydrocarbon solvent to the human body may be low and the excellent dissolving power of the hydrocarbon solvent may be maintained.

An aspect of the present invention provides a hydrocarbon solvent composition having excellent dissolving power and drying property, no odor, and low harmfulness to a human body.

**Claims**

1. A hydrocarbon solvent composition comprising:

   80 wt% or more of a $C_7$ saturated hydrocarbon component and 1 wt% or less of an aromatic component, based on the total weight of the hydrocarbon solvent composition,
   wherein the hydrocarbon solvent composition satisfies the following Equation 1:

   [Equation 1]

   $$5 < \frac{W_{IC7}}{W_{NC7}} < 100$$

   wherein $W_{IC7}$ is a content of $C_7$ isoparaffin, based on the total weight of the hydrocarbon solvent composition, and $W_{NC7}$ is a content of $C_7$ n-paraffin, based on the total weight of the hydrocarbon solvent composition.

2. The hydrocarbon solvent composition of claim 1, further comprising 1 wt% or less of a $C_5$ saturated hydrocarbon component, based on the total weight of the hydrocarbon solvent composition.

3. The hydrocarbon solvent composition of claim 1, further comprising 1 wt% or less of a $C_8$ saturated hydrocarbon component, based on the total weight of the hydrocarbon solvent composition.

4. The hydrocarbon solvent composition of claim 1, further comprising 30 wt% or more and 45 wt% or less of a C7 cyclic saturated hydrocarbon component, based on the total weight of the hydrocarbon solvent composition.

5. The hydrocarbon solvent composition of claim 1, further comprising 5 wt% or less of $C_7$ n-paraffin, based on the total weight of the hydrocarbon solvent composition.

6. The hydrocarbon solvent composition of claim 1, wherein the hydrocarbon solvent composition further satisfies the following Equation 2:

   [Equation 2]

   $$1 \leq \frac{W_{IC}}{W_N} \leq 1.4$$

   wherein $W_{IC}$ is a content of isoparaffin, based on the total weight of the hydrocarbon solvent composition, and $W_N$ is a content of a cyclic saturated hydrocarbon component, based on the total weight of the hydrocarbon solvent composition.

7. The hydrocarbon solvent composition of claim 1, further comprising less than 1 ppm of sulfur, based on the total weight of the hydrocarbon solvent composition.

8. The hydrocarbon solvent composition of claim 1, wherein the hydrocarbon solvent composition includes 80 wt% or more and 99.5 wt% or less of the $C_7$ saturated hydrocarbon component, 0.0001 wt% or more and 1 wt% or less of the aromatic component, and 0.4 wt% or more and 19 wt% or less of the remaining components other than the $C_7$ saturated hydrocarbon component and the aromatic component, based on the total weight of the hydrocarbon solvent composition.

9. The hydrocarbon solvent composition of claim 1, wherein the hydrocarbon solvent composition includes 80 wt% or more and 99.5 wt% or less of the $C_7$ saturated hydrocarbon component, 0.0001 wt% or more and 1 wt% or less of the aromatic component, 0.001 wt% or more and 1 wt% or less of a $C_5$ saturated hydrocarbon component, 0.001 wt% or more and 1 wt% or less of a $C_8$ saturated hydrocarbon component, and 0.1 wt% or more and 17 wt% or less of a $C_6$ saturated hydrocarbon component, based on the total weight of the hydrocarbon solvent composition.

10. The hydrocarbon solvent composition of claim 1, wherein the hydrocarbon solvent composition has an aniline point of 70°C or less.

11. The hydrocarbon solvent composition of claim 1, wherein the hydrocarbon solvent composition has an evaporation rate of 25 to 45 when an evaporation rate of n-butyl acetate (n-BuAc) is set to 100 according to ASTM D3539.

**Patentansprüche**

1. Kohlenwasserstoff-Lösungsmittelzusammensetzung, aufweisend:

   80 Gew.-% oder mehr einer gesättigten $C_7$-Kohlenwasserstoffkomponente und 1 Gew.-% oder weniger einer aromatischen Komponente, basierend auf dem Gesamtgewicht der Kohlenwasserstoff-Lösungsmittelzusammensetzung,
   wobei die Kohlenwasserstoff-Lösungsmittelzusammensetzung die folgende Gleichung 1 erfüllt:

   [Gleichung 1]

   $$5 < \frac{W_{IC7}}{W_{NC7}} < 100$$

   wobei $W_{IC7}$ ein Gehalt an $C_7$-Isoparaffin ist, basierend auf dem Gesamtgewicht der Kohlenwasserstoff-Lösungsmittelzusammensetzung, und $W_{NC7}$ ein Gehalt an $C_7$-n-Paraffin ist, basierend auf dem Gesamtgewicht der Kohlenwasserstoff-Lösungsmittelzusammensetzung.

2. Kohlenwasserstoff-Lösungsmittelzusammensetzung nach Anspruch 1, ferner aufweisend 1 Gew.-% oder weniger einer gesättigten Cs-Kohlenwasserstoffkomponente basierend auf dem Gesamtgewicht der Kohlenwasserstoff-Lösungsmittelzusammensetzung.

3. Kohlenwasserstoff-Lösungsmittelzusammensetzung nach Anspruch 1, ferner aufweisend 1 Gew.-% oder weniger einer gesättigten Cs-Kohlenwasserstoffkomponente basierend auf dem Gesamtgewicht der Kohlenwasserstoff-Lösungsmittelzusammensetzung.

4. Kohlenwasserstoff-Lösungsmittelzusammensetzung nach Anspruch 1, ferner aufweisend 30 Gew.-% oder mehr und 45 Gew.-% oder weniger einer zyklisch gesättigten C7-Kohlenwasserstoffkomponente basierend auf dem Gesamtgewicht der Kohlenwasserstoff-Lösungsmittelzusammensetzung.

5. Kohlenwasserstoff-Lösungsmittelzusammensetzung nach Anspruch 1, ferner aufweisend 5 Gew.-% oder weniger $C_7$ n-Paraffin basierend auf dem Gesamtgewicht der Kohlenwasserstoff-Lösungsmittelzusammensetzung.

6. Kohlenwasserstoff-Lösungsmittelzusammensetzung nach Anspruch 1, wobei die Kohlenwasserstoff-Lösungsmittelzusammensetzung ferner die folgende Gleichung 2 erfüllt:

[Gleichung 2]

$$1 \leq \frac{W_{IC}}{W_N} < 1,4$$

wobei $W_{IC}$ ein Gehalt an Isoparaffin ist, basierend auf dem Gesamtgewicht der Kohlenwasserstoff-Lösungsmittelzusammensetzung, und $W_N$ ein Gehalt einer cyclischen gesättigten Kohlenwasserstoffkomponente ist, basierend auf dem Gesamtgewicht der Kohlenwasserstoff-Lösungsmittelzusammensetzung.

7. Kohlenwasserstoff-Lösungsmittelzusammensetzung nach Anspruch 1, ferner aufweisend weniger als 1 ppm Schwefel basierend auf dem Gesamtgewicht der Kohlenwasserstoff-Lösungsmittelzusammensetzung.

8. Kohlenwasserstoff-Lösungsmittelzusammensetzung nach Anspruch 1, wobei die Kohlenwasserstoff-Lösungsmittelzusammensetzung 80 Gew.-% oder mehr und 99,5 Gew.-% oder weniger der gesättigten $C_7$-Kohlenwasserstoffkomponente, 0,0001 Gew.-% oder mehr und 1 Gew.-% oder weniger der aromatischen Komponente und 0,4 Gew.-% oder mehr und 19 Gew.-% oder weniger der übrigen Komponenten, welche nicht die gesättigte $C_7$-Kohlenwasserstoffkomponente und die aromatischen Komponente sind, basierend auf dem Gesamtgewicht der Kohlenwasserstoff-Lösungsmittelzusammensetzung aufweist.

9. Kohlenwasserstoff-Lösungsmittelzusammensetzung nach Anspruch 1, wobei die Kohlenwasserstoff-Lösungsmittelzusammensetzung 80 Gew.-% oder mehr und 99,5 Gew.-% oder weniger der gesättigten $C_7$-Kohlenwasserstoffkomponente, 0,0001 Gew.-% oder mehr und 1 Gew.-% oder weniger der aromatischen Komponente, 0,001 Gew.-% % oder mehr und 1 Gew.-% oder weniger einer gesättigten $C_5$-Kohlenwasserstoffkomponente, 0,001 Gew.-% oder mehr und 1 Gew.-% oder weniger einer gesättigten $C_8$-Kohlenwasserstoffkomponente und 0,1 Gew.-% oder mehr und 17 Gew.-% oder weniger einer gesättigten $C_6$-Kohlenwasserstoffkomponente basierend auf dem Gesamtgewicht der Kohlenwasserstoff-Lösungsmittelzusammensetzung aufweist.

10. Kohlenwasserstoff-Lösungsmittelzusammensetzung nach Anspruch 1, wobei die Kohlenwasserstoff-Lösungsmittelzusammensetzung einen Anilinpunkt von 70°C oder weniger aufweist.

11. Kohlenwasserstoff-Lösungsmittelzusammensetzung nach Anspruch 1, wobei die Kohlenwasserstoff-Lösungsmittelzusammensetzung eine Verdampfungsrate von 25 bis 45 aufweist, wenn eine Verdampfungsrate von n-Butylacetat (n-BuAc) gemäß ASTM D3539 auf 100 eingestellt ist.

## Revendications

1. Composition de solvant hydrocarburé comprenant :

au moins 80 % en poids d'un constituant hydrocarbure saturé en $C_7$ et au plus 1 % en poids d'un constituant aromatique, par rapport au poids total de la composition de solvant hydrocarburé, la composition de solvant hydrocarburé satisfaisant à l'Équation 1 suivante :

[Équation 1]

$$5 < \frac{W_{IC7}}{W_{NC7}} < 100$$

dans laquelle $W_{IC7}$ est une teneur en isoparaffine $C_7$, par rapport au poids total de la composition de solvant hydrocarburé, et $W_{NC7}$ est une teneur en n-paraffine $C_7$, par rapport au poids total de la composition de solvant hydrocarburé.

2. La composition de solvant hydrocarburé de la revendication 1, comprenant en outre au plus 1 % en poids d'un constituant hydrocarbure saturé en $C_5$, par rapport au poids total de la composition de solvant hydrocarburé.

3. La composition de solvant hydrocarburé de la revendication 1, comprenant en outre au plus 1 % en poids d'un constituant hydrocarbure saturé en $C_8$, par rapport au poids total de la composition de solvant hydrocarburé.

4.  La composition de solvant hydrocarburé de la revendication 1, comprenant en outre entre au moins 30 % en poids et au plus 45 % en poids d'un constituant hydrocarbure saturé cyclique en $C_7$, par rapport au poids total de la composition de solvant hydrocarburé.

5.  La composition de solvant hydrocarburé de la revendication 1, comprenant en outre au plus 5 % en poids de n-paraffine $C_7$, par rapport au poids total de la composition de solvant hydrocarburé.

6.  La composition de solvant hydrocarburé de la revendication 1, dans laquelle la composition de solvant hydrocarburé satisfait en outre à l'Équation 2 suivante :

[Équation 2]

$$1 \leq \frac{W_{IC}}{W_N} < 1,4$$

dans laquelle $W_{IC}$ est une teneur en isoparaffine, par rapport au poids total de la composition de solvant hydrocarburé, et $W_N$ est une teneur en un constituant hydrocarbure saturé cyclique, par rapport au poids total de la composition de solvant hydrocarburé.

7.  La composition de solvant hydrocarburé de la revendication 1, comprenant en outre moins d'1 ppm de soufre, par rapport au poids total de la composition de solvant hydrocarburé.

8.  La composition de solvant hydrocarburé de la revendication 1, dans laquelle la composition de solvant hydrocarburé comprend entre au moins 80 % en poids et au plus 99,5 % en poids du constituant hydrocarbure saturé en $C_7$, entre au moins 0,0001 % en poids et au plus 1 % en poids du constituant aromatique, et entre au moins 0,4 % en poids et au plus 19 % en poids des constituants restants autres que le constituant hydrocarbure saturé en $C_7$ et le constituant aromatique, par rapport au poids total de la composition de solvant hydrocarburé.

9.  La composition de solvant hydrocarburé de la revendication 1, dans laquelle la composition de solvant hydrocarburé comprend entre au moins 80 % en poids et au plus 99,5 % en poids du constituant hydrocarbure saturé en $C_7$, entre au moins 0,0001 % en poids et au plus 1 % en poids du constituant aromatique, entre au moins 0,001 % en poids et au plus 1 % en poids d'un constituant hydrocarbure saturé en $C_5$, entre au moins 0,001 % en poids et au plus 1 % en poids d'un constituant hydrocarbure saturé en $C_8$, et entre au moins 0,1 % en poids et au plus 17 % en poids d'un constituant hydrocarbure saturé en $C_6$, par rapport au poids total de la composition de solvant hydrocarburé.

10. La composition de solvant hydrocarburé de la revendication 1, dans laquelle la composition de solvant hydrocarburé présente un point d'aniline égal ou inférieur à 70 °C.

11. La composition de solvant hydrocarburé de la revendication 1, dans laquelle la composition de solvant hydrocarburé présente un taux d'évaporation compris entre 25 et 45 lorsqu'un taux d'évaporation de l'acétate de n-butyle (n-BuAc) est réglé sur 100 selon ASTM D3539.